**Europäisches Patentamt**

**European Patent Office**

**Office européen des brevets**

(19)

(11) Veröffentlichungsnummer: **0 212 040**
**B1**

(12) **EUROPÄISCHE PATENTSCHRIFT**

(45) Veröffentlichungstag der Patentschrift:
**23.05.90**

(21) Anmeldenummer: **85890174.7**

(22) Anmeldetag: **05.08.85**

(51) Int. Cl.⁵: **A61L 2/00, A61K 35/16**

(54) **Verfahren zur Herstellung von Präparationen auf Basis von Blutgerinnungsfaktoren.**

(43) Veröffentlichungstag der Anmeldung:
**04.03.87 Patentblatt 87/10**

(45) Bekanntmachung des Hinweises auf die Patenterteilung:
**23.05.90 Patentblatt 90/21**

(84) Benannte Vertragsstaaten:
**AT BE CH DE FR GB IT LI LU NL SE**

(56) Entgegenhaltungen:
**EP-A- 0 094 611**
**EP-A- 0 142 059**
**EP-A- 0 159 311**
**WO-A-82/03871**
**DE-A- 3 247 150**

**Die Akte enthält technische Angaben, die nach dem Eingang der Anmeldung eingereicht wurden und die nicht in dieser Patentschrift enthalten sind.**

(73) Patentinhaber: **IMMUNO Aktiengesellschaft für chemisch-medizinische Produkte, Industriestrasse 72, A-1220 Wien(AT)**

(72) Erfinder: **Elsinger, Friedrich, Dr., Eduard Kleingasse 29, A-1130 Wien(AT)**

(74) Vertreter: **Wolfram, Gustav, Dipl.-Ing., Schwindgasse 7 P.O. Box 205, A-1041 Wien(AT)**

## Beschreibung

Die Erfindung betrifft ein Verfahren zur Herstellung einer Faktor VIII (Antihämophilfaktor, AHF)-Präparation unter Hitzebehandlung in trockenem Zustand zur Inaktivierung von etwa vorhandenen Viren.

Es sind bereits verschiedene Verfahren bekannt, die sich mit der Hitzeinaktivierung von vermehrungsfähigen Krankheitserregern in Blutprodukten befassen. So ist in der PCT-Anmeldung WO 82/03 871 ein Verfahren zur Behandlung von relativ hitzestabile Blutgerinnungsenzyme enthaltenden Zubereitungen beschrieben, wobei diese zur Inaktivierung vorhandener Viren im trockenen Zustand bei einem Wassergehalt von weniger als 5 Gew.% (0,05) Wasser erhitzt werden; diese Behandlung kann gegebenenfalls unter Anwendung einer Stickstoffatmosphäre oder unter Vakuum vorgenommen werden. Weiter ist in der EP-A2 0 094 611 ein Verfahren zur Behandlung einer Faktor VIII enthaltenden Komposition im lyophilisierten Zustand beschrieben, wobei die Komposition unter atmosphärischen Bedingungen auf eine Temperatur von wenigstens 60°C erhitzt wird.

In der EP-A2 0 142 059 ist ein Verfahren zur Hitzebehandlung von Plasmaproteinen beschrieben, wobei ein viruskontaminiertes Plasmaprotein im trockenen Zustand in Gegenwart einer sauren Aminosäure und einer basischen Aminosäure als Stabilisierungsmittel, z.B. zusammen mit einer Kombination von L-Glutaminsäure und L-Lysin auf mindestens 60°C, gegebenenfalls unter Schutzgas, erhitzt wird. Stabilisierungsmittel haben jedoch den Nachteil, daß sie nicht nur eine Schutzwirkung auf die Plasmaproteine ausüben, sondern auch auf die Viren, die inaktiviert werden sollen, und damit die Inaktivierung verzögern bzw. schärfere Maßnahmen erfordern, um eine vollständige Virusinaktivierung zu erreichen.

Bei den zum Stand der Technik gehörenden Verfahren, bei welchen Faktor VIII-Präparationen im trockenen Zustand der Hitzeinaktivierungsbehandlung unterworfen werden, ergibt sich die Schwierigkeit, gleichermaßen eine Sicherheit gegen Übertragung von Hepatitis-Viren und anderen pathogenen Viren zu gewährleisten und die Erhaltung der biologischen Aktivität des behandelten Blutproduktes sicherzustellen. Diese Schwierigkeit tritt deshalb auf, weil die zu behandelnde Faktor VIII-Präparation hitzeempfindlich ist. Bei den notwendigen langen Erhitzungszeiten und hohen Temperaturen, die erforderlich sind, um eine vollständige Virusinaktivierung zu erreichen, treten bei der Behandlung von Faktor VIII-Präparationen Verluste der biologischen Aktivität auf.

Die Erfindung bezweckt die Vermeidung der geschilderten Nachteile und Schwierigkeiten und stellt sich die Aufgabe, ein Virus-Inaktivierungsverfahren für eine Faktor VIII (Antihämophilfaktor, AHF)-Präparation durch Erhitzen in trockenem Zustand zu schaffen, wobei unter Anwendung von verhältnismäßig langen Behandlungszeiten bzw. unter vergleichbar hoher Temperatur eine zuverlässige Inaktivierung von vermehrungsfähigen filtrierbaren Krankheitserregern bei gleichzeitig weitgehender Erhaltung der biologischen Aktivität gewährleistet wird.

Diese Aufgabe wird bei einem Verfahren der eingangs bezeichneten Art dadurch gelöst, daß bei Anwendung hoher Temperaturen und langer Behandlungszeiten – entsprechend einer Temperatur von 90°C und einer Behandlungsdauer von 30 Stunden – die Hitzebehandlung der Präparation in Gegenwart eines sauerstofffreien inerten Schutzgases oder unter Vakuum, jedoch in Abwesenheit von Stabilisierungsmitteln, durchgeführt wird, um die biologische Aktivität der Faktor VIII-Präparation weitgehend, nämlich mit einer Restaktivität von mindestens 0,60, zu erhalten.

Das erfindungsgemäße Verfahren wird im folgenden näher erläutert:

Da die notwendigen Untersuchungen über Hepatitis-Virusinaktivierung in Blutprodukten nicht sofort beim Menschen vorgenommen werden können und Schimpansen nur in ungenügender Zahl zur Verfügung stehen, erfolgt erfindungsgemäß die Bewertung der Wirksamkeit der Inaktivierung mit Hilfe von Modellviren. Am Beispiel von Bakteriophagen T4 als Modellvirus konnte gezeigt werden, daß bei der Hitzebehandlung einer Faktor VIII-Präparation in Gegenwart von Stickstoff bzw. unter Vakuum die biologische Aktivität weitgehend erhalten wird, obwohl zur vollständigen Inaktivierung der Bakteriophagen T4 lange Erhitzungszeiten bei hoher Temperatur angewendet werden.

Das erfindungsgemäße Inaktivierungsverfahren, die Herstellung einer Präparation, die erreichten Wirkungen und die Überlegenheit gegenüber den bekannten Verfahren ohne Inertgasatmosphäre sind im folgenden Beispiel und der Tabelle näher erläutert:

Beispiel:

a) Herstellung einer Faktor VIII-Präparation

6660 ml frisch gefrorenes Plasma wurden bei 0°C bis +4°C aufgetaut. Das entstandene Kryopräzipitat wurde durch Zentrifugieren abgetrennt und in 700 ml Trinatriumcitrat-Lösung, die 0,05 mg Natriumpentosansulfat pro ml und 30 Einheiten Aprotinin pro ml enthielt, gelöst. Der pH-Wert der Lösung wurde auf 6,3 und die Temperatur auf +4°C gestellt. Der entstandene Niederschlag wurde durch Zentrifugieren abgetrennt und verworfen.

Durch Zugabe von Äthanol bis zu einer Konzentration von 0,08 wurde die Faktor VIII-haltige Fraktion ausgefällt. Der entstandene Niederschlag wurde durch Zentrifugieren abgetrennt und in einem Glycin-Citrat-NaCl-Puffer gelöst.

b) Inaktivierung eines Modellvirus

Die in beschriebener Weise erhaltene Faktor VIII-Lösung wurde auf 50 mg Protein/ml eingestellt. Die Lösung wurde mit einer Suspension des Bakteriophagen T4 in Kulturmedium für Escherichia coli 11303 bzw. mit virusfreiem Kulturmedium versetzt und gefriergetrocknet. Geschlossene Behälter mit gefriergetrockneten Faktor VIII-Proben — mit und ohne Virus — wurden bei einer Temperatur von 90°C verschieden lang erhitzt. Je zwei Proben wurden vor dem Erhitzen und zu bestimmten Zeiten während des Erhitzungsvorganges zur Messung des Virustiters einerseits und der Reaktivität anderseits entnommen.

Die Bestimmung des Virustiters wurde in folgender Weise durchgeführt:

Das Faktor VIII enthaltende Lyophilisat wurde nach der Hitzebehandlung in Wasser gelöst und mit 1 mM $MgCl_2$ im Verhältnis 1:10 seriell verdünnt. Eine Mischung von 3 ml 0,7% Bacto-Agar®, 43 bis 45°C, 100 µl Suspension von E.coli in Flüssigmedium und 200 µl der bakteriophagenhaltigen Probe bzw. Verdünnung wurden durchgemischt und rasch auf einer Nähragarplatte (ATCC 129) ausgegossen. Nach Inkubation über Nacht bei 37°C wurden mit Hilfe eines Zählgerätes die durch vermehrungsfähige Viruspartikel erzeugten Plaques im konfluenten Zellrasen gezählt (PFU, plaque-forming units). Die Ergebnisse wurden als $\log_{10}$ PFU ausgedrückt und sind aus der Tabelle ersichtlich.

c) Bestimmung der Restaktivität an virusfreien Proben

Die Bestimmung der Restaktivität an Faktor VIII erfolgte mit Hilfe des Thromboplastin-Bildungs-Tests (2-Stufen-Test). Die Faktor VIII-Restaktivität wurde berechnet durch Bildung des Quotienten aus der Faktor VIII-Aktivität der erhitzten Probe und der Faktor VIII-Aktivität der entsprechenden nicht erhitzten Probe.

Das beschriebene Inaktivierungsverfahren wurde zum Vergleich der Ergebnisse unter verschiedener Atmosphäre durchgeführt, u.zw. unter Luft, Stickstoff und Vakuum; die ermittelten Virustiter und die Restaktivität Faktor VIII nach dem Erhitzen während verschiedener Zeiten, nämlich nach 1, 3, 10 und 30 h sind in der Tabelle angeführt. Während die vollständige Virusinaktivierung unabhängig von der Erhitzungs-Atmosphäre erreicht wird, ist die Restaktivität an Faktor VIII nach 30 Stunden Erhitzung bei 90°C in Stickstoffatmosphäre (0,67) bzw. unter Vakuum (0,66), also mehr als 0,60 und damit doppelt so hoch wie bei Erhitzung in Luft-Atmosphäre (0,30).

EP 0 212 040 B1

<u>Erhitzen einer Faktor VIII-Präparation unter Luft, Stickstoff und Vakuum</u>

| Wasser-gehalt | Tempe-ratur °C | Atmo-sphäre | Bakteriophagen T4-Virustiter (Log$_{10}$ PFU) nach Erhitzen während | | | | | | Restaktivität Faktor VIII nach Erhitzen während | | | | |
|---|---|---|---|---|---|---|---|---|---|---|---|---|---|
| | | | 0 | 1 | 3 | 10 | 30 | h | 1 | 3 | 10 | 30 | h |
| 0,006 | 90 | Luft | 3,9 | 3,0 | 2,3 | 1,1 | 0 | | 0,84 | 0,75 | 0,57 | 0,30 | |
| 0,006 | 90 | Stick-stoff | 4,1 | 3,3 | 2,5 | 1,2 | 0 | | 0,98 | 0,91 | 0,81 | 0,67 | |
| 0,006 | 90 | Vakuum | 3,8 | 3,0 | 2,2 | 1,1 | 0 | | 0,96 | 0,88 | 0,76 | 0,66 | |

**Patentansprüche**

Verfahren zur Herstellung einer Faktor VIII (Antihämophilfaktor, AF)-Präparation unter Hitzebehandlung in trockenem Zustand zur Inaktivierung von etwa vorhandenen Viren, dadurch gekennzeichnet, daß bei Anwendung hoher Temperaturen und langer Behandlungszeiten – entsprechend einer Temperatur von 90°C und einer Behandlungsdauer von 30 Stunden – die Hitzebehandlung der Präparation in Gegenwart eines sauerstofffreien inerten Schutzgases oder unter Vakuum, jedoch in Abwesenheit von Stabilisierungsmitteln, durchgeführt wird, um die biologische Aktivität der Faktor VIII-Präparation weitgehend, nämlich mit einer Restaktivität von mindestens 0,60, zu erhalten.

**Claims**

A method of producing a Factor VIII (antihaemophilic factor, AHF) preparation by heat treatment in the dry state to inactivate possibly present viruses, characterised in that, at the application of elevated temperatures and extended treatment times – corresponding to a temperature of 90°C and a treatment time of 30 hours – the heat treatment of the preparation is carried out in the presence of an oxygen-free inert protective gas or under vacuum, yet in the absence of stabilising agents, in order to largely preserve the biologic activity of the Factor VIII preparation, i.e., a residual activity of at least 0.60.

**Revendications**

Procédé pour la fabrication d'une préparation de facteur VIII (facteur antihémophilique, AHF) avec traitement par la chaleur à l'état sec pour l'inactivation de virus éventuellement présents, caractérisé en ce que le traitement par la chaleur de la préparation est mis en œuvre en présence d'un gaz inerte exempt d'oxygène ou sous vide, mais en l'absence d'agents stabilisants, avec application de températures élevées et de longues durées de traitement – correspondant à une température de 90°C et une durée de traitement de 30 heures – afin de conserver largement l'activité biologique de la préparation de facteur VIII, à savoir avec une activité résiduelle d'au moins 0,60.